# EUROPÄISCHE PATENTSCHRIFT

(11) **EP 3 329 903 B1**
(45) Veröffentlichungstag und Bekanntmachung des Hinweises auf die Patenterteilung: **05.02.2020**
(21) Anmeldenummer: 17202748.4
(22) Anmeldetag: 21.11.2017
(51) Int. Cl.: A61K 8/37, A61K 8/46, A61K 8/60, A61Q 19/10, A61K 8/04, A61K 8/06

(54) **KOSMETISCHER SCHAUM AUS EINER O/W-EMULSION MIT EINEM VERESTERUNGSPRODUKTE AUS GLYCERIN MIT DREI IDENTISCHEN FETTSÄUREN**
COSMETIC FOAM MADE FROM AN O/W EMULSION WITH AN ESTERIFICATION PRODUCT MADE FROM GLYCEROL WITH THREE IDENTICAL FATTY ACIDS
MOUSSE COSMÉTIQUE À BASE D'UNE ÉMULSION HUILE DANS EAU CONTENANT UN PRODUIT D'ESTÉRIFICATION À LA GLYCÉRINE DOTÉ DES TROIS ACIDES GRAS IDENTIQUES

(30) Priorität: 02.12.2016 DE 102016224030
(43) Veröffentlichungstag der Anmeldung: 06.06.2018
(73) Patentinhaber: Beiersdorf AG, 20253 Hamburg (DE)
(72) Erfinder: Johns, Nicole, 22958 Kuddewörde (DE); Max, Heiner, 22529 Hamburg (DE); Möllgaard, Svenja Lena, 22337 Hamburg (DE); Schäfer, Jessica, 22113 Oststeinbek (DE)

(56) Entgegenhaltungen:
- EP-A1- 1 377 271
- EP-A1- 2 020 221
- DE-A1- 10 139 581
- DE-T2- 69 703 712

## Beschreibung

Die vorliegende Erfindung betrifft ein kosmetisches Produkt aufweisend einen Druckbehälter mit Entnahmeventil enthaltend eine O/W-Emulsion umfassend ein oder mehr Veresterungsprodukte aus Glycerin mit drei identischen linearen und/oder verzweigten Fettsäuren mit einer Alkylkette mit 12 bis 20 Kohlenstoffatomen und mindestens ein die O/W-Emulsion aufschäumendes und/oder austragendes Treibmittel, dadurch gekennzeichnet, dass die O/W-Emulsion Tripalmitin, Tristearin und/oder Triisostearin enthält.

Schön und attraktiv auszusehen ist ein Bedürfnis von vielen Menschen. Oftmals gilt dabei eine reine und gepflegte Haut als Schönheitsideal. Um diesem zu entsprechen, werden die verschiedensten kosmetischen Produkte zur täglichen Reinigung und Pflege der Haut eingesetzt.

Die Reinigung der Haut dient dazu Schmutz, Schweiß und Reste abgestorbener Körperzellen, die als Nährstoffe für Krankheitserreger und Parasiten aller Art dienen können, zu entfernen. Dazu werden oftmals kosmetische Zubereitungen, die als Emulsion formuliert sind, eingesetzt. Unter Emulsionen versteht man im Allgemeinen heterogene Systeme, die aus zwei nicht oder nur begrenzt mischbaren Flüssigkeiten bestehen, wobei eine der beiden Flüssigkeiten in Form feinster Tröpfchen in der anderen Flüssigkeit dispergiert ist. Mit dem bloßen Auge erscheint eine Emulsion als homogen. Sind beide Flüssigkeiten Wasser und Öl, und liegt das Öl als fein verteilte Tröpfchen im Wasser vor, so handelt es sich um eine Öl-in-Wasser-Emulsion (O/W-Emulsion). Liegt hingegen das Wasser als fein verteilte Tröpfchen im Öl vor, so handelt es sich um eine Wasser-in-ÖI-Emulsion (W/O-Emulsion).

Wird nachfolgend der Begriff Haut verwendet so bezieht sich dieser ausschließlich auf die menschliche Haut.

Beim Waschvorgang wird die kosmetische Zubereitung zunächst auf die Haut appliziert und dort mit den Händen oder einem Hilfsmittel, wie einem Pad, Tuch oder ähnlichem, verrieben. Anschließend wird die kosmetische Zubereitung zusammen mit den gelösten Schmutz, Schweiß oder Resten abgestorbener Körperzellen entweder mit einem Hilfsmittel abgenommen oder mit Wasser abgespült.

Dabei sind beim Anwender insbesondere kosmetische Zubereitungen beliebt, die sich vor oder bei Anwendung auf der Haut aufschäumen lassen und eine große Menge Schaum produzieren.

Eine Möglichkeit eine gut schäumende kosmetische Zubereitung bereitzustellen besteht darin schäumende und waschaktive Tenside einzusetzen. Da waschaktive Tenside allerdings oftmals auch reizend auf die Haut wirken können, sind kosmetische Zubereitungen wünschenswert, die mit nur einem minimalen Anteil an Tensiden gleichzeitig eine effektive Hautreinigung und Schaumbildung ermöglichen.

Alternativ zu den klassischen Emulsionen, die zur Reinigung der Haut im flüssigen Zustand aus dem Vorratsbehältnis entnommen und auf der Haut aufgetragen werden, gibt es eine geringe Anzahl an Produkten, bei denen die Emulsion zur Anwendung auf der Haut mit Hilfe von Treibgas aufgeschäumt wird. Dabei wird ein Schaum mit moussiger Textur erhalten, der dem Anwender nicht nur Spaß bei Auftragen auf die Haut bereitet, sondern auch zumeist als gut auf der Haut verteilbar wahrgenommen wird.

Nachteilig am Stand der Technik ist jedoch der Umstand, dass aufgeschäumte O/W-Emulsionen oftmals die Haut austrocknen und/oder die Haut unzureichend befeuchten. Dieses ist insbesondere problematisch für Personen die chronisch an trockener Haut leiden. Dementsprechend muss oftmals auf die Anwendung von aufgeschäumten Emulsionen verzichtet werden.

Hautbefeuchtung oder Befeuchtung der Haut bedeutet im Sinne der vorliegenden Offenbarung, dass der Anteil von Wasser, das in der menschlichen Haut gespeichert wird, nach Anwendung der kosmetischen Zubereitung auf der Haut erhöht wird.

Daher war es Aufgabe der vorliegenden Erfindung ein kosmetisches Produkt bereitzustellen, welches einen Schaum aus einer O/W-Emulsion zu erhalten, der nach Anwendung auf der Haut den Anteil an in der Haut gespeichertem Wasser erhöht und so verbesserte Pflegeeigenschaften aufweist.

Nachteilig am Stand der Technik war außerdem, dass aufgeschäumte kosmetische Zubereitungen in Form von O/W-Emulsionen oftmals unzureichende sensorische Eigenschaften aufweisen. So ist es vom Verbraucher gewünscht, dass der Schaum fein-blasig und cremig (feinporig) ist und nicht umgehend nach dem aufschäumen zerfällt. Des Weiteren ist es nachteilig, wenn die erhaltenen Schäume eine unzureichende Haftbarkeit aufweisen und so von der Haut schnell ablaufen oder abrutschen. Dabei kann es bei Gesichtsreinigungsprodukten unter Umständen dazu kommen, dass der Schaum in die Augen läuft, was abhängig vom Produkt, ein brennendes Gefühl hervorrufen könnte.

Es war daher die Aufgabe der vorliegenden Erfindung eine aufgeschäumte kosmetische Zubereitung mit einem sensorisch attraktiven und somit fein-blasigen (feinporig), cremigen, und/oder gleichmäßigen Schaum bereitzustellen, der über einen längeren Zeitraum strukturell stabil bleibt, beziehungsweise seine äußere Form nicht verändert, und/oder eine gute Haftbarkeit aufweist.

Überraschend gelöst wurde die Aufgabe durch die vorliegende Erfindung.

Die Erfindung ist ein kosmetisches Produkt aufweisend einen Druckbehälter mit Entnahmeventil enthaltend
a) eine O/W-Emulsion umfassend ein oder mehr Veresterungsprodukte aus Glycerin mit drei identischen linearen und/oder verzweigten Fettsäuren mit einer Alkylkette mit 12 bis 20 Kohlenstoffatomen, und
b) mindestens ein die O/W-Emulsion aufschäumendes und/oder austragendes Treibmittel, dadurch gekennzeichnet, dass die O/W-Emulsion Tripalmitin, Tristearin und/oder Triisostearin enthält.

Auch Gegenstand der Erfindung ist die Verwendung des erfindungsgemäßen kosmetischen Produkts zur Bereitstellung eines Schaums, welcher eine verbesserte Feinporigkeit aufweist. Das heißt der Schaum ist weniger grobblasig.

Auch Gegenstand der Erfindung ist die Verwendung des erfindungsgemäßen kosmetischen Produkts zur Bereitstellung eines Schaums, welcher eine erhöhte Cremigkeit aufweist.

Auch Gegenstand der Erfindung ist die Verwendung des erfindungsgemäßen kosmetischen Produkts zur Bereitstellung eines Schaums, welcher eine erhöhte strukturelle Stabilität aufweist. Das heißt der Schaum fällt nach Austragen und/oder aufschäumen mit dem Treibmittel weniger schnell zusammen.

WO 2014012699 A2 offenbart in Beispiel 55 ein kosmetisches Sonnenschutzspray enthaltend Triisostearin. Im Unterschied zur vorliegenden Erfindung sind alle Zubereitungen aus WO 2014012699 A2 als W/O-Emulsion formuliert. Zudem wird dem Fachmann in WO 2014012699 A2 kein Hinweis auf die vorliegende Erfindung gegeben.

Auch WO 2014012705 A2 offenbart kosmetische Sonnenschutzsprays auf Basis von W/O-Emulsionen, die laut Beschreibung neben einer Vielzahl andere Ölkomponenten Triisostearin enthalten können. Es wird kein Hinweis auf die vorliegende Erfindung gegeben. Das erfindungsgemäße kosmetische Produkt bildet nach ausbringen einen überraschend feinblasigen, gleichmäßigen, stabilen und cremigen Schaum.

Alle nachfolgend aufgeführten Gewichtsprozentangaben (Gew.-%) beziehen sich, sofern nicht anders angegeben, jeweils auf das Gesamtgewicht der O/W-Emulsion.

Der Ausdruck "frei von" bedeutet im Sinne der vorliegenden Offenbarung, dass der Anteil der jeweiligen Substanz kleiner 0,05 Gew.-% ist. Dadurch wird gewährleistet, dass Einschleppungen oder Verunreinigungen mit diesen Stoffen nicht als erfindungsgemäß "frei von" mitumfasst werden.

Es ist erfindungsgemäß von Vorteil, wenn die erfindungsgemäße O/W-Emulsion ein oder mehr Veresterungsprodukte aus Glycerin mit drei identischen linearen und/oder verzweigten Fettsäuren mit einer Alkylkette mit 12 bis 20 Kohlenstoffatomen in einem Anteil von 0,01 Gew.-% bis 15 Gew.-%, bevorzugt 0,5 Gew.-% bis 10 Gew.-% und insbesondere bevorzugt 1 Gew.-% bis 5 Gew.-% enthält.

Erfindungsgemäße Veresterungsprodukte aus Glycerin mit drei identischen linearen und/oder verzweigten Fettsäuren Alkylkette mit 12 bis 20 Kohlenstoffatomen sind unter den INCI-Namen Tripalmitin, Tristearin und Triisostearin bekannt. Insbesondere bevorzugt ist Triisostearin.

Eine besonders bevorzugte Ausführungsform der vorliegenden Erfindung ist dadurch gekennzeichnet, dass die O/W-Emulsion Triisostearin in einem Anteil von 0,01 Gew.-% bis 15 Gew.-%, bevorzugt 0,5 Gew.-% bis 10 Gew.-% und insbesondere bevorzugt 1 Gew.-% bis 5 Gew.-% enthält und die O/W-Emulsion frei von weiteren Veresterungsprodukten von Glycerin mit drei identischen linearen und/oder verzweigten Fettsäuren mit einer Alkylkette mit 12 bis 20 Kohlenstoffatomen ist.

Die erfindungsgemäßen Veresterungsprodukte aus Glycerin mit drei identischen linearen und/oder verzweigten Fettsäuren mit einer Alkylkette mit 12 bis 20 Kohlenstoffatomen enthalten keine weiteren Alkoxy-Einheiten, wie beispielsweise Ethylenoxid oder Propylenoxid Gruppen. Es ist erfindungsgemäß von Vorteil, wenn der Druckbehälter des kosmetischen Produkts befüllt ist mit 90 Gew.-% bis 96 Gew.-% O/W-Emulsion und 4 Gew.-% bis 10 Gew.-% Treibmittel, bezogen auf das Gesamtgewicht aller im Druckbehälter enthaltenen Inhaltsstoffe. Dabei ist es erfindungsgemäß bevorzugt, wenn der Anteil der O/W-Emulsion 92 Gew.-% bis 94 Gew.-% und der Anteil des Treibmittels 6 Gew.-% bis 8 Gew.-%, bezogen auf das Gesamtgewicht der Inhaltsstoffe, beträgt. Im Druckbehälter sind keine weiteren Inhaltsstoffe enthalten.

Als Treibmittel werden erfindungsgemäß die Gase der Gruppe Butan, Iso-Butan, Pentan, IsoPentan, Stickstoff und Kohlenstoffdioxid bezeichnet. Dabei ist es erfindungsgemäß von Vorteil, wenn als Treibmittel Butan, Iso-Butan und/oder Propan gewählt werden. Nicht zu verwenden sind Fluorchlorkohlenwasserstoffe, welche die Umwelt beeinträchtigen. Es ist insbesondere von Vorteil, wenn neben Butan, Iso-Butan und/oder Propan keine weiteren Treibmittel enthalten sind. Das Mischungsverhältnis dieser Gase variiert dabei je nach Druckstufe, zum Beispiel:
Druckstufe 2,7 bar: 60 Gew.-% Butan, 20 Gew.-% Propan und 20 Gew.-% Iso-Butan;
Druckstufe 3,0 bar: 5,3 Gew.-% Butan, 15,3 Gew.-% Propan und 79,4 Gew.-% Iso-Butan;
Druckstufe 3,5 bar: 5 Gew.-% Butan, 23 Gew.-% Propan und 72 Gew.-% Iso-Butan;
jeweils bezogen auf das Gesamtgewicht des gesamten Treibmittels.

Erfindungsgemäß bevorzugt sind die Druckstufen 2,7 bar, 3,0 bar und 3,5 bar.

Besonders bevorzugt sind die Druckstufen 3,0 bar und 3,5 bar.

Des Weiteren ist es vorteilhaft, wenn die erfindungsgemäße O/W-Emulsion ein oder mehrere Emulgatoren gewählt aus der Gruppe der Verbindungen Glycerylstearatcitrat, Cetearylsulfat (insbesondere Natriumcetylsulfat), Glycerylstearat, Cetylphosphat (insbesondere Kaliumcetylphosphat), Natriumstearoylglutamat und/oder Polyglyceryl-10 Stearat enthält.

Erfindungsgemäß bevorzugt ist es dabei entweder Glycerylstearatcitrat oder eine Mischung aus Cetearylsulfat (insbesondere Natriumcetylsulfat) und Glycerylstearat einzusetzen. Wird Glycerylstearatcitrat oder eine Mischung aus Cetearylsulfat (insbesondere Natriumcetylsulfat) und Glycerylstearat eingesetzt, so wird ein überraschend feinporiger und stabiler Schaum erhalten.

Erfindungsgemäß besonders bevorzugt wird dabei selbstemulgierendes Glycerylmonostearat mit der INCI Glyceryl Monostearate SE eingesetzt.

Wird die erfindungsgemäße O/W-Emulsion mit Glycerylstearatcitrat emulgiert, so ist es erfindungsgemäß bevorzugt, diesen Emulgator in einer Konzentration von 0,1 Gew.-% bis 5 Gew.-% einzusetzen.

Wird die erfindungsgemäße O/W-Emulsion mit Cetearylsulfat emulgiert, so ist es erfindungs-gemäß bevorzugt, diesen Emulgator in einer Konzentration von 0,1 Gew.-% bis 2 Gew.-% einzusetzen.

Wird die erfindungsgemäße O/W-Emulsion mit Glycerylstearat SE emulgiert, so ist es erfindungs-gemäß bevorzugt, diesen Emulgator in einer Konzentration von 0,1 Gew.-% bis 5 Gew.-% einzusetzen.

Wird die erfindungsgemäße O/W-Emulsion mit Cetylphosphat emulgiert, so ist es erfindungsgemäß bevorzugt, diesen Emulgator in einer Konzentration von 0,1 bis 5 Gew.-% einzusetzen.

Wird die erfindungsgemäße O/W-Emulsion mit Natriumstearoylglutamat emulgiert, so ist es erfindungsgemäß bevorzugt, diesen Emulgator in einer Konzentration von 0,1 Gew.-% bis 2 Gew.-% einzusetzen.

Wird die erfindungsgemäße O/W-Emulsion mit Polyglyceryl-10 Stearat emulgiert, so ist es erfindungsgemäß bevorzugt, diesen Emulgator in einer Konzentration von 0,1 Gew.-% bis 5 Gew.-% einzusetzen.

Ferner ist es vorteilhaft im Sinne der vorliegenden Erfindung, wenn die O/W-Emulsion ein oder mehrere Tenside enthält. Durch Einsatz von Tenside werden besonders feinporige und stabile Schäume erhalten.

Enthält die O/W-Emulsion Tenside, so ist es erfindungsgemäß von Vorteil, wenn der Anteil der Tenside 0,05 Gew.-% bis 5 Gew.-%, bevorzugt 0,1 Gew.-% bis 3 Gew.-% und insbesondere bevorzugt 0,5 Gew.-% bis 1,5 Gew.-% beträgt.

Wenn die O/W-Emulsion Tenside enthält, ist es zudem vorteilhaft, wenn die Tenside gewählt werden aus den nicht-ionischen Tensiden. Dabei ist es insbesondere vorteilhaft, wenn neben nicht-ionischen Tensiden keine weiteren Tenside in der O/W-Emulsion enthalten sind.

Erfindungsgemäß bevorzugte nicht-ionische Tenside sind Alkylglucoside mit Alkylresten mit einer Kettenlänge von 6 bis 20 Kohlenstoffatomen. Unter diesen werden insbesondere bevorzugt Decylglucosid und/oder Laurylglucosid gewählt. Decylglucosid ist unter anderem unter der Bezeichnung Plantaren® 2000 N UP von der Fa. BASF erhältlich. Laurylglucosid kann unter dem Handelsnamen Plantaren® 1200 N UP von der Fa. BASF bezogen werde. Sowohl mit Decylglucosid als auch mit Laurylglucosid wurden besonders ferinporige und stabile Schäume erhalten. Des Weiteren werden diese Schäume als besonders cremig empfunden.

Erfindungsgemäß vorteilhafte Ausführungsformen der Erfindung sind dadurch gekennzeichnet, dass die erfindungsgemäße O/W-Emulsion Phenoxyethanol, Benzethoniumchlorid und/oder Ethylhexylglycerin enthält.

Enthält die O/W-Emulsion Phenoxyethanol, so ist es erfindungsgemäß von Vorteil, wenn der Anteil an Phenoxyethanol 0,1 Gew.-% bis 1 Gew.-% beträgt.

Enthält die O/W-Emulsion Benzethoniumchlorid, so ist es erfindungsgemäß von Vorteil, wenn der Anteil an Benzethoniumchlorid 0,01 Gew.-% bis 0,4 Gew.-% beträgt.

Enthält die O/W-Emulsion Ethylhexylglycerin, so ist es erfindungsgemäß von Vorteil, wenn der Anteil an Ethylhexylglycerin 0,1 Gew.-% bis 2 Gew.-% beträgt.

Es ist erfindungsgemäß vorteilhaft, wenn die erfindungsgemäße O/W-Emulsion dadurch gekennzeichnet ist, dass die erfindungsgemäße O/W-Emulsion Benzylalkohol, Methylparaben und/oder Ethylparaben enthält.

Enthält die erfindungsgemäße O/W-Emulsion Benzylalkohol, so ist es erfindungsgemäß bevorzugt, diesen Stoff in einem Anteil von 0,1 Gew.-% bis 2 Gew.-% einzusetzen.

Enthält die erfindungsgemäße O/W-Emulsion Methylparaben, so ist es erfindungsgemäß bevorzugt, diesen Stoff in einem Anteil von 0,1 Gew.-% bis 1 Gew.-% einzusetzen.

Enthält die erfindungsgemäße O/W-Emulsion Ethylparaben, so ist es erfindungsgemäß bevorzugt, diesen Stoff in einem Anteil von 0,1 Gew.-% bis 1 Gew.-% einzusetzen.

Erfindungsgemäß vorteilhafte Ausführungsformen der vorliegenden Erfindung sind dadurch gekennzeichnet, dass die Fettphase der O/W-Emulsion ein oder mehrere Fettalkohole enthält, wobei Stearylalkohol, Cetylalkohol und/oder deren Mischungen bevorzugt gewählt werden.

Der erfindungsgemäß vorteilhafte Anteil von Stearylalkohol, Cetylalkohol und/oder deren Mischungen in der erfindungsgemäßen O/W-Emulsion beträgt 0,1 Gew.-% bis 2 Gew.-%.

Des Weiteren sind erfindungsgemäß vorteilhafte Ausführungsformen der vorliegenden Erfindung dadurch gekennzeichnet, dass die Fettphase der O/W-Emulsion ein oder mehrere Ester von linearen oder verzweigten, gesättigten oder ungesättigten Alkoholen mit 2 bis 6 Kohlenstoffatomen mit linearen oder verzweigten, gesättigten oder ungesättigten Fettsäuren mit 12 bis 18 Kohlenstoffatomen enthält. Diese Ester werden bevorzugt gewählt aus der Gruppe Isopropylmyristat, Isopropylpalmitat, Isopropylstearat und/oder Isopropylisostearat.

Die erfindungsgemäß vorteilhafte Anteil von Isopropylmyristat, Isopropylpalmitat, Isopropylstearat und/oder Isopropylisostearat beträgt 2 Gew.-% bis 8 Gew.-%.

Ferner sind erfindungsgemäß vorteilhafte Ausführungsformen der vorliegenden Erfindung dadurch gekennzeichnet, dass Dicaprylylether in einem Anteil von 1,5 Gew.-% bis 5 Gew.-% in der erfindungsgemäßen O/W-Emulsion enthalten ist.

Es ist erfindungsgemäß von Vorteil, wenn der Anteil der Fettphase der O/W-Emulsion 7,5 Gew.-% bis 15 Gew.-% beträgt.

Vorteilhaft kann die erfindungsgemäße O/W-Emulsion Glycerin enthalten. Es ist dabei erfindungsgemäß bevorzugt, wenn Glycerin in einem Anteil von 5 Gew.-% bis 15 Gew.-% enthalten ist.

Des Weiteren ist es vorteilhaft, wenn die erfindungsgemäße O/W-Emulsion Methylpropanediol enthält. Besonders vorteilhaft ist dabei Methylpropanediol in einem Anteil von 0,5 Gew.-% bis 4 Gew.-% enthalten.

Es ist erfindungsgemäß vorteilhaft, wenn die erfindungsgemäße O/W-Emulsion einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivat, Glycyrrhiza Inflata Root Extract, Magnolienextrakt enthält.

Erfindungsgemäß vorteilhafte Ausführungsformen der vorliegenden Erfindung sind dadurch gekennzeichnet, dass die erfindungsgemäße O/W-Emulsion frei von Seifen ist.

Erfindungsgemäß werden Seifen als Natrium- oder Kaliumsalze von Fettsäuren verstanden.

Da Seifen oftmals zur Stabilisierung von Schäumen, wie beispielsweise bei Rasierschaum, eingesetzt werden, war es überraschend, dass das erfindungsgemäße kosmetische Produkt der vorteilhaften Ausführungsform stabile Schäume bildet. Seifenfreie kosmetische Produkte haben ferner den Vorteil, dass diese ein signifikant reduziertes Hautreizungspotential aufweisen.

Die erfindungsgemäße O/W-Emulsion hat vorteilhaft einen pH-Wert im Bereich von 5 bis 7.

Des Weiteren ist es vorteilhaft, wenn die erfindungsgemäße O/W-Emulsion frei von UV-Filtern ist.

Im Sinne der vorliegenden Erfindung werden als UV-Filter die Substanzen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; -Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid bezeichnet.

Ferner ist es vorteilhaft, wenn die erfindungsgemäße O/W-Emulsion frei von Verdickungsmittel ist. Als Verdickungsmittel werden im Sinne der vorliegenden Erfindung Carbomere, Polysaccharide und Crosspolymere bezeichnet. Der Einsatz von Verdickungsmitteln würde zu Schäumen mit unvorteilhaften sensorischen Eigenschaften führen.

Erfindungsgemäß vorteilhafte Ausführungsformen der vorliegenden Erfindung sind dadurch gekennzeichnet, dass die erfindungsgemäße O/W-Emulsion frei von Mineralöl und/oder Silikonöl ist. Sowohl Mineralöle als auch Silikonöle können die Sensorik der aufgeschäumten kosmetischen O/W-Emulsion beeinträchtigen.

Zur Anwendung wird das erfindungsgemäße kosmetische Produkt durch Schütteln des Druckbehälters zuerst durchmischt und anschließend über das Entnahmeventil entnommen und auf die Haut appliziert. Bei Entnahme aus dem Druckbehälter wird ein Schaum bzw. eine aufgeschäumte kosmetische Zubereitung erhalten.

Als Druckbehälter mit Entnahmeventil können die üblichen für kosmetische Zwecke bekannten Aerosoldosen-Systeme eingesetzt werden. Bevorzugt Druckbehälter mit Sprüh- bzw. Schaumköpfe eingesetzt. Ein Beispiel eines geeigneten Schaumkopfes ist der APTAR Accessories Foam Upright S25 Foam.

Erfindungsgemäß ist daher auch ein Verfahren zur Anwendung des erfindungsgemäßen kosmetischen Produkts auf der Haut, welches dadurch gekennzeichnet ist, dass die Inhaltsstoffe des Druckbehälters durch Schütteln in des Druckbehälters zuerst durchmischt und anschließend über das Entnahmeventil entnommen und auf die Haut appliziert wird.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **Bsp.1** | **Bsp.2** | **Bsp.3** | **Bsp. 4** | **Vgl. Bsp.** |
|---|---|---|---|---|---|
| **O/W-Emulsion:** | | | | | |
| Panthenol (75% in Wasser) | 1,4 | 1,3 | 1,4 | 1,5 | 1,0 |
| Dicaprylyl Ether | 3 | 3 | 2,8 | 3,1 | 3 |
| Isopropyl Palmitate | 5 | 2,5 | 5 | 5 | 5 |
| Isopropylstearat | 0 | 2,5 | 0 | 0 | 0 |
| Triisostearin | 2 | 2 | 2 | 0 | 0 |
| Tripalmitin | 0 | 0 | 0 | 2 | 0 |
| Glyceryl Stearate SE | 2,4 | 2,4 | 2,4 | 2,4 | 2,4 |
| Sodium Cetearyl Sulfate | 0,15 | 0,15 | 0,15 | 0,15 | 0,15 |
| Parfum | 0,25 | 0,25 | 0,25 | 0,25 | 0,25 |
| Glycerin (99% in Wasser) | 9,3 | 9,3 | 9,3 | 9,3 | 9,3 |
| Methylpropanediol | 2 | 0 | 2 | 2 | 2 |
| Citric Acid | 0,03 | 0,03 | 0,03 | 0,03 | 0,03 |
| Phenoxyethanol | 0,6 | 0,6 | 0,6 | 0,6 | 0,6 |
| Benzethonium Chloride | 0,02 | 0 | 0,02 | 0,02 | 0,02 |
| Methylparaben | 0 | 0,3 | 0 | 0 | 0 |
| Cetearyl Alcohol | 1 | 1 | 1 | 1 | 1 |
| Lauryl Glucoside (48-55% in Wasser) | 1,1 | 1,1 | 0 | 0 | 1,1 |
| Decyl Glucoside (48-55% in Wasser) | 0 | 0 | 1,1 | 1,1 | 0 |
| Aqua | ad 100 | ad 100 | ad 100 | ad 100 | ad 100 |
| | | | | | |

| **Befüllung in 200 mL Dose:** | | | | | |
|---|---|---|---|---|---|
| O/W-Emulsion | 94% | 94% | 94% | 94% | 94% |
| Treibmittel | Propan/Butan/iso-Butan | Propan/Butan/iso-Butan | Propan/Butan/iso-Butan | Propan/Butan/iso-Butan | Propan/Butan/iso-Butan |
| Druckstufe | 3,0 bar | 3,0 bar | 3,0 bar | 3,0 bar | 3,0 bar |
| | | | | | |
| Sensorik des Schaums | cremiger, stabiler, feinblasiger Schaum | cremiger, stabiler, feinblasiger Schaum | cremiger, stabiler, feinblasiger Schaum | cremiger, stabiler, feinblasiger Schaum | weniger cremig, grobblasiger Schaum |

Die Beispiele Bsp. 1, Bsp. 2, Bsp. 3 und Bsp. 4 stellen erfindungsgemäße Beispiele da, während Vgl. Bsp. ein nicht erfindungsgemäßes Beispiel ist.

## Patentansprüche

1. Kosmetisches Produkt aufweisend einen Druckbehälter mit Entnahmeventil enthaltend
a) eine O/W-Emulsion umfassend ein oder mehr Veresterungsprodukte aus Glycerin mit drei identischen linearen und/oder verzweigten Fettsäuren mit einer Alkylkette mit 12 bis 20 Kohlenstoffatomen, und
b) mindestens ein die O/W-Emulsion aufschäumendes und/oder austragendes Treibmittel,
**dadurch gekennzeichnet, dass** die O/W-Emulsion Tripalmitin, Tristearin und/oder Triisostearin enthält.

2. Kosmetisches Produkt nach Anspruch 1, **dadurch gekennzeichnet, dass** die O/W-Emulsion ein oder mehrere Veresterungsprodukte aus Glycerin mit drei identischen linearen und/oder verzweigten Fettsäuren mit einer Alkylkette mit 12 bis 20 Kohlenstoffatomen in einem Anteil von 0,01 Gew.-% bis 15 Gew.-%, bevorzugt 0,5 Gew.-% bis 10 Gew.-% und insbesondere bevorzugt 1 Gew.-% bis 5 Gew.-%, bezogen auf das Gesamtgewicht der O/W-Emulsion, enthält.

3. Kosmetisches Produkt nach einem der vorhergehenden Ansprüche, **dadurch gekennzeichnet, dass** die O/W-Emulsion Tripalmitin, Tristearin und Triisostearin enthält, wobei Triisotearin bevorzugt ist.

4. Kosmetisches Produkt nach einem der vorhergehenden Ansprüche, **dadurch gekennzeichnet, dass** der Druckbehälter des kosmetischen Produkts befüllt ist mit 90 Gew.-% bis 96 Gew.-% O/W-Emulsion und 4 Gew.-% bis 10 Gew.-% Treibmittel, bezogen auf das Gesamtgewicht aller im Druckbehälter enthaltenen Inhaltsstoffe.

5. Kosmetisches Produkt nach einem der vorhergehenden Ansprüche, **dadurch gekennzeichnet, dass** als Treibmittel Butan, Iso-Butan und/oder Propan gewählt werden.

6. Kosmetisches Produkt nach einem der vorhergehenden Ansprüche, **dadurch gekennzeichnet, dass** die O/W-Emulsion ein oder mehrere Emulgatoren gewählt aus der Gruppe der Verbindungen Glycerylstearatcitrat, Cetearylsulfat (insbesondere Natriumcetylsulfat), Glycerylstearat, Cetylphosphat (insbesondere Kaliumcetylphosphat), Natriumstearoylglutamat und/oder Polyglyceryl-10 Stearat, enthält.

7. Kosmetisches Produkt nach einem der vorhergehenden Ansprüche, **dadurch gekennzeichnet, dass** die O/W-Emulsion ein oder mehrere Tenside enthält, welche vorteilhaft aus den nicht-ionischen Tensiden gewählt werden.

8. Kosmetisches Produkt nach Anspruch 6, **dadurch gekennzeichnet, dass** die Tenside gewählt werden aus den Alkylglucosiden mit Alkylresten mit einer Kettenlänge von 6 bis 20 Kohlenstoffatomen.

9. Kosmetisches Produkt nach einem der vorhergehenden Ansprüche, **dadurch gekennzeichnet, dass** die O/W-Emulsion Phenoxyethanol, Benzethoniumchlorid und/oder Ethylhexylglycerin enthält.

10. Kosmetisches Produkt nach einem der vorhergehenden Ansprüche, **dadurch gekennzeichnet, dass** die O/W-Emulsion Benzylalkohol, Methylparaben und/oder Ethylparaben enthält.

11. Kosmetisches Produkt nach einem der vorhergehenden Ansprüche, **dadurch gekennzeichnet, dass** die O/W-Emulsion Stearylalkohol, Cetylalkohol und/oder deren Mischungen enthält.

12. Kosmetisches Produkt nach einem der vorhergehenden Ansprüche, **dadurch gekennzeichnet, dass** die Fettphase der O/W-Emulsion ein oder mehrere Ester von linearen oder verzweigten, gesättigten oder ungesättigten Alkoholen mit 2 bis 6 Kohlenstoffatomen mit linearen oder verzweigten, gesättigten oder ungesättigten Fettsäuren mit 12 bis 18 Kohlenstoffatomen enthält.

13. Kosmetisches Produkt nach einem der vorhergehenden Ansprüche, **dadurch gekennzeichnet, dass** die O/W-Emulsion Dicaprylylether in einem Anteil von 1,5 Gew.-% bis 5 Gew.-%, bezogen auf das Gesamtgewicht der O/W-Emulsion, enthält.

14. Kosmetisches Produkt nach einem der vorhergehenden Ansprüche, **dadurch gekennzeichnet, dass** die O/W-Emulsion frei von Seifen ist.

15. Verwendung von eines kosmetischen Produkts gemäß einem der Ansprüche 1 bis 14 zur Bereitstellung eines Schaums, welcher eine verbesserte Feinporigkeit, erhöhte Cremigkeit und/oder eine erhöhte strukturelle Stabilität aufweist.

## Claims

1. Cosmetic product comprising a pressure vessel with a withdrawal valve, containing
a) an O/W emulsion comprising one or more esterification products of glycerol with three identical linear and/or branched fatty acids having an alkyl chain having 12 to 20 carbon atoms, and
b) at least one blowing agent that foams up and/or discharges the O/W emulsion,
**characterized in that** the O/W emulsion contains tripalmitin, tristearin and/or triisostearin.

2. Cosmetic product according to Claim 1, **characterized in that** the O/W emulsion contains one or more esterification products of glycerol with three identical linear and/or branched fatty acids having an alkyl chain having 12 to 20 carbon atoms in a proportion of 0.01% by weight to 15% by weight, preferably 0.5% by weight to 10% by weight and especially preferably 1% by weight to 5% by weight, based on the total weight of the O/W emulsion.

3. Cosmetic product according to either of the preceding claims, **characterized in that** the O/W emulsion contains tripalmitin, tristearin and triisostearin, preferably triisostearin.

4. Cosmetic product according to any of the preceding claims, **characterized in that** the pressure vessel of the cosmetic product has been filled with 90% by weight to 96% by weight of O/W emulsion and 4% by weight to 10% by weight of blowing agent, based on the total weight of all ingredients present in the pressure vessel.

5. Cosmetic product according to any of the preceding claims, **characterized in that** the blowing agent selected is butane, isobutane and/or propane.

6. Cosmetic product according to any of the preceding claims, **characterized in that** the O/W emulsion contains one or more emulsifiers selected from the group of compounds glyceryl stearate citrate, cetearyl sulfate (especially sodium cetyl sulfate), glyceryl stearate, cetyl phosphate (especially potassium cetyl phosphate), sodium stearoyl glutamate and/or polyglycerol-10 stearate.

7. Cosmetic product according to any of the preceding claims, **characterized in that** the O/W emulsion contains one or more surfactants that are advantageously selected from the nonionic surfactants.

8. Cosmetic product according to Claim 6, **characterized in that** the surfactants are selected from the alkyl glucosides having alkyl radicals having a chain length of 6 to 20 carbon atoms.

9. Cosmetic product according to any of the preceding claims, **characterized in that** the O/W emulsion contains phenoxyethanol, benzethonium chloride and/or ethylhexylglycerol.

10. Cosmetic product according to any of the preceding claims, **characterized in that** the O/W emulsion contains benzyl alcohol, methyl paraben and/or ethyl paraben.

11. Cosmetic product according to any of the preceding claims, **characterized in that** the O/W emulsion contains stearyl alcohol, cetyl alcohol and/or mixtures thereof.

12. Cosmetic product according to any of the preceding claims, **characterized in that** the lipid phase of the O/W emulsion contains one or more esters of linear or branched, saturated or unsaturated alcohols having 2 to 6 carbon atoms with linear or branched, saturated or unsaturated fatty acids having 12 to 18 carbon atoms.

13. Cosmetic product according to any of the preceding claims, **characterized in that** the O/W emulsion contains dicaprylyl ether in a proportion of 1.5% by weight to 5% by weight, based on the total weight of the O/W emulsion.

14. Cosmetic product according to any of the preceding claims, **characterized in that** the O/W emulsion is free of soaps.

15. Use of a cosmetic product according to any of Claims 1 to 14 for providing a foam having an improved fine pore content, elevated creaminess and/or elevated structural stability.

## Revendications

1. Produit cosmétique comprenant un contenant sous pression muni d'une soupape de prélèvement, contenant :
a) une émulsion H/E comprenant un ou plusieurs produits d'estérification de glycérine avec trois acides gras linéaires et/ou ramifiés identiques contenant une chaîne alkyle de 12 à 20 atomes de carbone, et
b) au moins un agent gonflant qui fait mousser et/ou qui décharge l'émulsion H/E,
**caractérisé en ce que** l'émulsion H/E contient de la tripalmitine, de la tristéarine et/ou de la triisostéarine.

2. Produit cosmétique selon la revendication 1, **caractérisé en ce que** l'émulsion H/E contient un ou plusieurs produits d'estérification de glycérine avec trois acides gras linéaires et/ou ramifiés identiques contenant une chaîne alkyle de 12 à 20 atomes de carbone en une proportion de 0,01 % en poids à 15 % en poids, de préférence de 0,5 % en poids à 10 % en poids, et de manière particulièrement préférée de 1 % en poids à 5 % en poids, par rapport au poids total de l'émulsion H/E.

3. Produit cosmétique selon l'une quelconque des revendications précédentes, **caractérisé en ce que** l'émulsion H/E contient de la tripalmitine, de la tristéarine et de la triisostéarine, la triisostéarine étant préférée.

4. Produit cosmétique selon l'une quelconque des revendications précédentes, **caractérisé en ce que** le contenant sous pression du produit cosmétique est rempli avec 90 % en poids à 96 % en poids d'émulsion H/E et 4 % en poids à 10 % en poids d'agent gonflant, par rapport au poids total de tous les constituants contenus dans le contenant sous pression.

5. Produit cosmétique selon l'une quelconque des revendications précédentes, **caractérisé en ce que** le butane, l'isobutane et/ou le propane sont choisis en tant qu'agent gonflant.

6. Produit cosmétique selon l'une quelconque des revendications précédentes, **caractérisé en ce que** l'émulsion E/H contient un ou plusieurs émulsifiants choisis dans le groupe de composés constitué par le stéarate-citrate de glycéryle, le sulfate de cétéaryle (notamment le sulfate de cétyle sodique), le stéarate de glycéryle, le phosphate de cétyle (notamment le phosphate de cétyle potassique), le glutamate de stéaroyle sodique et/ou le stéarate de polyglycéryle-10.

7. Produit cosmétique selon l'une quelconque des revendications précédentes, **caractérisé en ce que** l'émulsion H/E contient un ou plusieurs tensioactifs, qui sont de préférence choisis parmi les tensioactifs non ioniques.

8. Produit cosmétique selon la revendication 6, **caractérisé en ce que** les tensioactifs sont choisis parmi les alkylglucosides contenant des radicaux alkyle ayant une longueur de chaîne de 6 à 20 atomes de carbone.

9. Produit cosmétique selon l'une quelconque des revendications précédentes, **caractérisé en ce que** l'émulsion H/E contient du phénoxyéthanol, du chlorure de benzéthonium et/ou de l'éthylhexylglycérine.

10. Produit cosmétique selon l'une quelconque des revendications précédentes, **caractérisé en ce que** l'émulsion H/E contient de l'alcool benzylique, du méthylparabène et/ou de l'éthylparabène.

11. Produit cosmétique selon l'une quelconque des revendications précédentes, **caractérisé en ce que** l'émulsion H/E contient de l'alcool stéarylique, de l'alcool cétylique et/ou leurs mélanges.

12. Produit cosmétique selon l'une quelconque des revendications précédentes, **caractérisé en ce que** la phase grasse de l'émulsion H/E contient un ou plusieurs esters d'alcools linéaires ou ramifiés, saturés ou insaturés, de 2 à 6 atomes de carbone, avec des acides gras linéaires ou ramifiés, saturés ou insaturés, de 12 à 18 atomes de carbone.

13. Produit cosmétique selon l'une quelconque des revendications précédentes, **caractérisé en ce que** l'émulsion H/E contient de l'éther dicaprylylique en une proportion de 1,5 % en poids à 5 % en poids, par rapport au poids total de l'émulsion H/E.

14. Produit cosmétique selon l'une quelconque des revendications précédentes, **caractérisé en ce que** l'émulsion H/E est exempte de savons.

15. Utilisation d'un produit cosmétique selon l'une quelconque des revendications 1 à 14 pour la préparation d'une mousse, qui présente une porosité fine améliorée, un caractère crémeux augmenté et/ou une stabilité structurale améliorée.
